# EUROPEAN PATENT SPECIFICATION

(11) **EP 2 332 917 B1**
(45) Date of publication and mention of the grant of the patent: **01.08.2012**
(21) Application number: 09014103.7
(22) Date of filing: 11.11.2009
(51) Int. Cl.: C07D 217/02, C07D 217/22, A61K 31/4709, A61P 35/00

(54) **Compounds for PIM kinase inhibition and for treating malignancy**
Verbindungen zur PIM-Kinasehemmung und zur Behandlung von Malignitäten
Composés pour l'inhibition de kinase PIM et pour le traitement des tumeurs

(43) Date of publication of application: 15.06.2011
(73) Proprietor: Sygnis Bioscience GmbH & Co. KG, 69120 Heidelberg (DE)
(72) Inventor: Schneider, Armin, 69118 Heidelberg (DE); Dittgen, Tanjew, 89073 Ulm (DE)
(74) Representative: Lahrtz, Fritz

(56) References cited:
- WO-A-2008/049919
- WO-A-2008/121687
- SATO TSUNEO ET AL: "Inhibition of human immunodeficiency virus type 1 replication by a bioavailable serine/threonine kinase inhibitor, fasudil hydrochloride" AIDS RESEARCH AND HUMAN RETROVIRUSES, MARY ANN LIEBERT, US, vol. 14, no. 4, 1 March 1998 (1998-03-01), pages 293-298, XP002548759 ISSN: 0889-2229

## Description

Oncological diseases belong to the worldwide leading causes of death. In the year 2004 they caused approximately 7.4 million death which was about 13 % of all death. The main types are lung, stomach, bowel, liver and breast cancer. Leukemia is one type of cancer in which the hematopoietic system is affected. They have been initially described by Rudolf Virchow who also coined the term. Subject to the progression of the disease, it is distinguished between acute and chronic leukemia. Acute leukemia are life-threatening diseases, causes death within few weeks or months if not treated. Chronic leukemia usually proceeds for years and exhibits hardly any symptoms during the initial stage. The main important types of leukemia are acute myelogenous leukemia (AML), acute lymphatic leukemia (ALL), chronic lymphatic leukemia (CLL) and chronic myelogenous leukemia (CML).

Lymphadenomas are tumors of the lymphatic system. Usually it is distinguished between two major types of lymphadenomas, namely the non-Hodgkin and the Hodgkin types. Non-Hodgkin lymphadenomas occur more than seven times more often than Hodgkin lymphadenomas. Non-Hodgkin lymphadenomas are classified into several different subtypes based on microscopic, genetic and immunologic characteristics. The less common Hodgkin lymphadenomas can be also subclassified using microscopic criteria. Subjects aged 15 to 25 years and aged more than 50 years represent the most frequently affected groups of the population.

The prostate carcinoma is the second leading tumor of the male population aged more than 50 years. It is a malignant tumor of the tissue of the prostate gland and in the majority of the cases it originates in the outer regions of the gland. The causes of this disease are so far mostly unknown. Genetic disposition seems to be relevant for the genesis of this disease. Therefore, the risk for developing a prostate carcinoma is regarded two times increased for men who's father or brother have had this carcinoma. The level of testosterone is considered to be an important factor since the tumor cells are dependent on the stimulus by androgens.

The PIM kinases are of high medical relevance. PIM kinases (PIM-1, -2, and -3) are highly conserved serine-threonine kinases belonging to the CAMK (calmodulin-dependent protein kinase-related) group that are key regulators in many signaling pathways implicated in cancer. PIM-1 was first identified with c-myc as a frequent pro viral insertion site in Moloney murine leukemia virus-induced T-cell lymphomas. When expressed, PIM kinases are strong survival factors and can induce progression of the cell cycle, inhibition of apoptosis, and modulation of other signal transduction pathways. Knockout mice for all three PIM genes develop normally but display reduced body size owing to decreased cell number in virtually all tissues. PIM kinases contribute to both cell proliferation and survival and thus provide a selective advantage in tumorigenesis. A number of proteins are phosphorylated by PIM kinases, such as transcriptional repressors (HP1), activators such as NFATc1 and c-Myb, co-activators (p100), as well as regulators of the cell cycle, such as p21WAF1/CIP1, Cdc25A phosphatase, and the kinase C-TAK1/MARK3/Par1A. PIM inhibitors can therefore induce cell death in cancer cells expressing PIM kinases and promote sensitivity of cancer cells to treatment with other targeted and chemotherapy drugs.

As PIM kinases contribute to many malignancies including prostate adenocarcinomas, pancreatic carcinoma, breast cancer, lung cancer, melanoma, liver carcinoma, gastric adenocarcinoma, diffuse large cell lymphomas, as well as several types of leukemias and other hematological malignancies, PIM kinase inhibition is useful for the treatment of a large number of malignancies. In particular, PIM kinase inhibition is useful for the treatment of leucemias ALL, CLL, AML, or CML, and Hodgkin- and Non-Hodgkin Lymphomas, mantle-cell lymphoma, Burkitt's lymphoma, and myeloproliferative disease (Amaravadi et al., J Clin Invest 2005, 115, 2618; Chiang et al, Int J Oral Maxillofac Surg. 2006, 35, 740; Dai et al. Acta Pharmacol Sin. 2005, 26, 364; Hu et al., J Clin Invest. 2009, 119, 362; Popivanova et al., Cancer Sci. 2007, 98, 321; Reiser-Erkan et al., Cancer Biol Ther. 2008, 7, 1352; Shah et al, Eu J Cancer 2008, 44, 2144; Tong et al., Bioorg Med Chem Lett. 2008, 18, 5206; Wang et al., J Vet Sci. 2001, 2, 167; Xia et al., J Med Chem. 2009, 52, 74; Zemskova et al., J Biol Chem. 2008, 283, 20635). Indeed a number of clinical trials are being initiated with compounds that target PIM kinases, such as SGI-1776. Therefore, compounds according to the invention which exhibit a particular specific inhibitory effect on PIM kinases are a new, highly attractive and desirable drug candidate. A specific advantage is the Pan-PIM activity, and the high specificity of the named compounds.

Besides, W02008121687 discloses compounds of Formula (I) thereof, which differ from the compounds of the present invention and which are receptor tyrosine inhibitors useful in the treatment of diseases mediated by class 3 and class 5 receptor tyrosine kinases, wherein particular compounds of said Formula (I) have also been found to be inhibitors of Pim-1.

In addition, W02008049919 discloses certain AGC kinase inhibitors, and particularly relates to ROCK inhibitors, compositions, comprising such inhibitors, and to uses of such inhibitors in the treatment and prophylaxis of disease.

In addition, a publication by Sato Tsuneo et al. ("Inhibition of human immunodeficiency virus type 1 replication by a bioavailable serine/threonine kinase inhibitor, fasudil hydrochloride", AIDS Res Hum Retroviruses. 1998 Mar 1;14(4):293-8) examines the effect of a newly developed serine/threonine kinase inhibitor, fasudil hydrochloride (FH), on the replication of HIV-1 and describes its efficacy in blocking HIV-1 replication in latently infected patients, although FH was initially developed as a compound that inhibited a myosin light chain kinase (MLCK) and had been approved for clinical use in the treatment of vasospasm after subarachnoid hemorrhage.

There is a need for additional PIM kinase inhibitors which are considered for treating oncological diseases, particularly of the hematopoietic system, the liver and the prostate gland.

Accordingly, the present invention relates to compounds which are particularly potent and highly specific inhibitors of PIM kinases and, thereby, are considered to be suitable for treating oncological diseases, particularly of the hematopoietic system, the liver and the prostate gland.

Specifically, the invention relates to compounds, methods and compositions as defined in the claims.

In one aspect, compounds of the following formula I are provided: 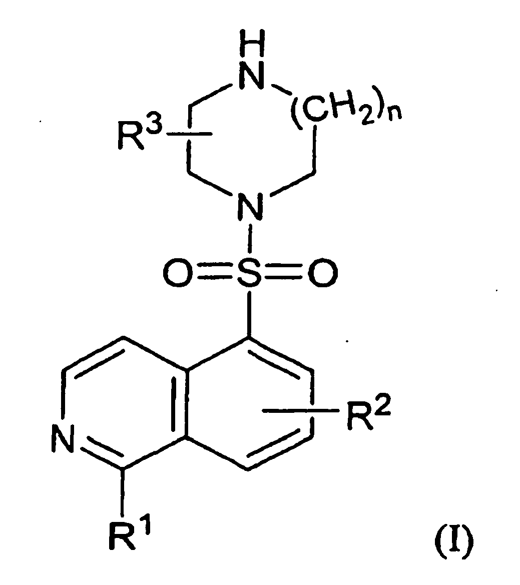 wherein R¹ is selected from the group consisting of a chlorine atom and a C₁₋₃ alkyl group, R² is a C₁₋₃ alkoxy group, such as a methoxy group, at position 6 or 8 of the isoquinoline moiety, R³ is a hydrogen or a C₁₋₃ alkyl group, and n is 1 or 2. In some embodiments, the compound 1-chloro-5-([1,4]diazepane-1-sulfonyl)-8-methoxy-isoquinoline or the compounds according to the formula 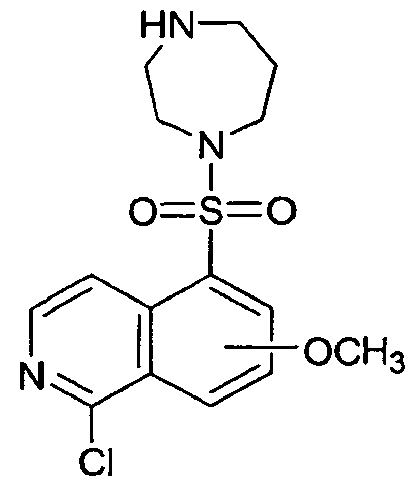 are excluded.

As used herein, the term "alkyl" refers to a straight or branched, saturated, aliphatic group having the number of carbon atoms indicated. Accordingly, "C₁₋₃ alkyl" refers to methyl, ethyl, n-propyl, and iso-propyl. In the same manner, "C₁₋₃ alkoxy" refers to methoxy, ethoxy, n-propoxy, and iso-propoxy.

One embodiment of the invention provides compounds of formula I, wherein R¹ is a chlorine atom. Another embodiment of the invention provides compounds of formula I, wherein R¹ is a C₁₋₃ alkyl group, such as a methyl group.

The compounds of formula I can also be salts, hydrates and solvates thereof.

In a further aspect, compounds of the following formula II are provided: 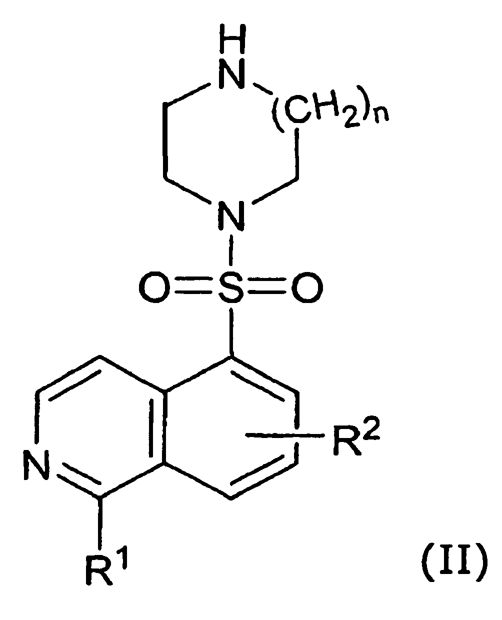 wherein R¹ is selected from the group consisting of a chlorine atom and a C₁₋₃ alkyl group, R² is a C₁₋₃ alkoxy group, such as a methoxy group, at position 6 or 8 of the isoquinoline moiety, and n is 1 or 2. In some embodiments, the compound 1-chloro-5-([1,4]diazepane-l-sulfonyl)-8-methoxy-isoquinoline or the compounds according to the formula 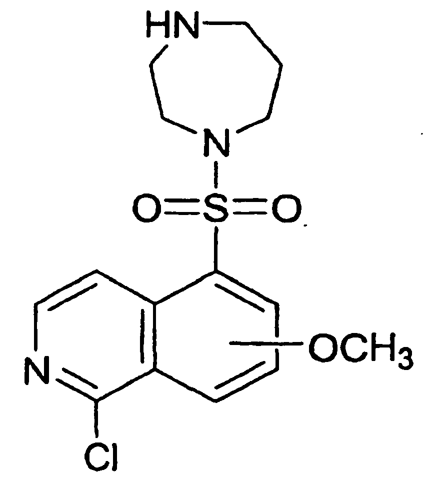 are excluded.

One embodiment of the invention provides compounds of formula II, wherein R¹ is a chlorine atom. Another embodiment of the invention provides compounds of formula II, wherein R¹ is a C₁₋₃ alkyl group, such as a methyl group.

The compounds of formula II can also be salts, hydrates and solvates thereof.

Exemplary compounds are selected from the group of 1-chloro-5-([1,4]diazepane-1-sulfonyl)-8-propoxy-isoquinoline, 1-chloro-5-(piperazin-1-sulfonyl)-8-methoxy-isoquinoline, 1-methyl-5-([1,4]diazepane-1-sulfonyl)-8-methoxy-isoquinoline, and 1-chloro-5-([1,4]diazepane-1-sulfonyl)-6-methoxy-isoquinoline. Additionally, these compounds include salts, hydrates and solvates thereof.

In a further embodiment of the invention, the compounds are selected from the group of 1-chloro-5-([1,4]diazepane-1-sulfonyl)-8-propoxy-isoquinoline, 1-chloro-5-(piperazin-1-sulfonyl)-8-methoxy-isoquinoline, and 1-methyl-5-([1,4]diazepane-1-sulfonyl)-8-methoxy-isoquinoline. Additionally, these compounds include salts, hydrates and solvates thereof.

In general, compounds of Formula I and II, and their salts and hydrates, are prepared using well-established methodologies and are based on the common knowledge of one skilled in the art. These are described, for instance, in U.S. Patent Nos. 4,678,783 and 5,942,505 and European Patent No. 187,371. More specific methodologies for representative compounds of the invention are presented in detail below.

The compounds of the invention can be used as selective and potent inhibitor for PIM kinases, particularly for PIM-1 kinase.

Said Compounds of the invention which exhibit a particular specific inhibitory effect on PIM kinases, such as PIM-1 kinase, therefore have broad therapeutic potential as a single agent as well as in combination with other agents (e.g. chemotherapeutic drugs and regimes known to anyone skilled in the art, such as Imatinib mesylate, mechlorethamine, cyclophosphamide, chlorambucil, cisplatin, carboplatin, oxaliplatin, azathioprine, mercaptopurine, doxorubicine, epirubicin, bleomycin, dactinomycin, vincristine, vinblastine, vinorelbine, vindesine, etoposide, teniposide, podophyllotoxin, paclitaxel, irinotecan, topotecan, melphalan, busulfan, capecitabine and combination thereof). They can be used for treating oncological diseases, particularly of the hematopoietic system, the liver and the prostate gland. Examples for such oncological diseases are ALL, CLL, AML, or CML, Hodgkin-Lymphoma, Non-Hodgkin Lymphoma and prostate carcinoma.

In another aspect, a compound of formula I or II is provided for use in a method for inhibiting a PIM kinase as defined in the claims. In one embodiment, said method is a method for treating PIM kinase related conditions in a subject, the method comprising administering to a patient in need thereof, a therapeutically effective amount of a compound of Formula I. In some embodiments, the condition is selected from the group consisting of ALL, CLL, AML, or CML, Hodgkin-Lymphoma and Non-Hodgkin Lymphoma.

In another a aspect, a compound of Formula I or II as specified above is provided for treating an oncological disease as defined in the claims.

In a further embodiment, said compound is for use in, a method for treating a an oncological disease in a subject suffering from said disease, comprising administering to the subject a therapeutically effective amount of a compound of Formula I or II as specified above. In one embodiment, said oncological disease is one of the hematopoietic system, the liver or the prostate gland. In another embodiment, said oncological disease is selected from the group consisting of acute myelogenous leukemia (AML), acute lymphatic leukemia (ALL), chronic lymphatic leukemia (CLL), chronic myelogenous leukemia (CML), Hodgkin lymphadenoma. Non-Hodgkin lymphadenoma, and prostate carcinoma.

The compounds of the present invention can be formulated in a variety of different manners known to one of skill in the art. Pharmaceutically acceptable carriers are determined in part by the particular composition being administered, as well as by the particular method used to administer the composition. Accordingly, there are a wide variety of suitable formulations of pharmaceutical compositions of the present invention (see, e.g., Remington's Pharmaceutical Sciences, 20t h ed., 2003, supra). Effective formulations include oral and nasal formulations, formulations for parenteral administration, and compositions formulated for with extended release.

Formulations suitable for oral administration can consist of (a) liquid solutions, such as an effective amount of a compound of the present invention suspended in diluents, such as water, saline or PEG 400; (b) capsules, sachets, depots or tablets, each containing a predetermined amount of the active ingredient, as liquids, solids, granules or gelatin; (c) suspensions in an appropriate liquid; (d) suitable emulsions; and (e) patches. The pharmaceutical forms can include one or more of lactose, sucrose, mannitol, sorbitol, calcium phosphates, corn starch, potato starch, microcrystalline cellulose, gelatin, colloidal silicon dioxide, talc, magnesium stearate, stearic acid, and other excipients, colorants, fillers, binders, diluents, buffering agents, moistening agents, preservatives, flavoring agents, dyes, disintegrating agents, and pharmaceutically compatible carriers. Lozenge forms can comprise the active ingredient in a flavor, e.g., sucrose, as well as pastilles comprising the active ingredient in an inert base, such as gelatin and glycerin or sucrose and acacia emulsions, gels, and the like containing, in addition to the active ingredient, carriers known in the art.

The pharmaceutical preparation typically is in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form. The composition can, if desired, also contain other compatible therapeutic agents. In some embodiments, the pharmaceutical preparations deliver the compounds of the invention in a sustained release formulation.

Pharmaceutical preparations useful in the present invention also include extended release formulations. In some embodiments, extended-release formulations useful in the present invention are described in U.S. Patent No. 6,699,508, which can be prepared according to U.S. Patent No. 7,125,567.

The pharmaceutical preparations are typically delivered to a mammal, including humans and non-human mammals. Non-human mammals treated using the present methods include domesticated animals (i.e., canine, feline, murine, rodentia, and lagomorpha) and agricultural animals (bovine, equine, ovine, porcine).

The compounds of the present invention can be administered as frequently as necessary, including hourly, daily, weekly or monthly. The compounds utilized in the pharmaceutical method of the invention are administered at the initial dosage of about 0.0001 mg/kg to about 1000 mg/kg daily. A daily dose range of about 0.01 mg/kg to about 500 mg/kg, or about 0.1 mg/kg to about 200 mg/kg, or about 1 mg/kg to about 100 mg/kg, or about 10 mg/kg to about 50 mg/kg, can be used. The dosages, however, may be varied depending upon the requirements of the patient, the severity of the condition being treated, and the compound being employed. For example, dosages can be empirically determined considering the type and stage of disease diagnosed in a particular patient. The dose administered to a patient, in the context of the present invention should be sufficient to effect a beneficial therapeutic response in the patient over time. The size of the dose also will be determined by the existence, nature, and extent of any adverse side-effects that accompany the administration of a particular compound in a particular patient. Determination of the proper dosage for a particular situation is within the skill of the practitioner. Generally, treatment is initiated with smaller dosages which are less than the optimum dose of the compound. Thereafter, the dosage is increased by small increments until the optimum effect under circumstances is reached. For convenience, the total daily dosage may be divided and administered in portions during the day, if desired. Doses can be given daily, or on alternate days, as determined by the treating physician. Doses can also be given on a regular or continuous basis over longer periods of time (weeks, months or years), such as through the use of a subdermal capsule, sachet or depot, implanted micro pump or via a patch.

The pharmaceutical compositions can be administered to the patient in a variety of ways, including topically, parenterally, intravenously, intradermally, subcutaneously, intramuscularly, colonically, rectally or intraperitoneally. In some embodiments, the pharmaceutical compositions are administered parenterally, topically, intravenously, intramuscularly, subcutaneously, orally, or nasally, such as via inhalation.

In practicing the methods and uses, respectively, in content of the present invention, the pharmaceutical compositions can be used alone, or in combination with other therapeutic or diagnostic agents. The additional drugs used in the combination protocols of the present invention can be administered separately or one or more of the drugs used in the combination protocols can be administered together, such as in an admixture. Where one or more drugs are administered separately, the timing and schedule of administration of each drug can vary. The other therapeutic or diagnostic agents can be administered at the same time as the compounds of the present invention, separately or at different times.

### Brief description of the figures:

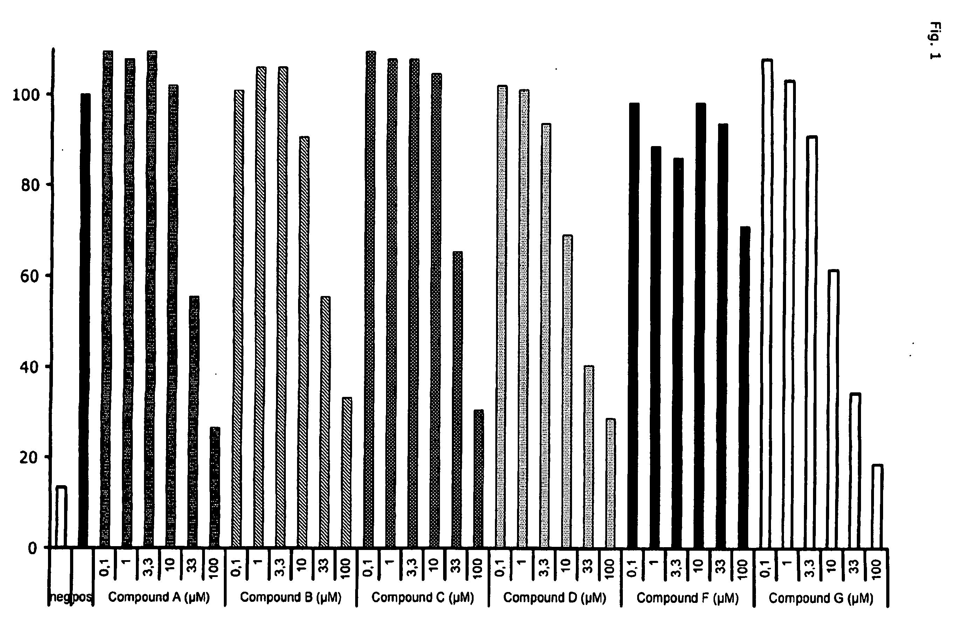

Fig. 1: The compounds 1-chloro-5-([1,4]diazepane-1-sulfonyl)-8-propoxy-isoquinoline (compound A), 1-chloro-5-(piperazin-1-sulfonyl)-8-methoxy-isoquinoline (compound B), 1-methyl-S-([1,4]diazepane-i-sulfonyl)-B-methoxy-isoquinoline (compound C), and 1-chloro-5-([1,4]diazepane-1-sulfonyl)-6-methoxy-isoquinoline (compound D) are potent PIM kinase inhibitors.
   In vitro assays of PIM-1 kinase activity (1.5 ng) in the present of increasing concentrations of the compounds A, B, C or D were performed as described within example 5. An assay without additional test compound served as positive control for the assay (pos) and an assay without PIM-1 kinase served as negative assay (neg). The compounds Fasudil (compound F) and potent PIM kinase inhibitor 1-thloro-8-methoxy-Fasudil (compound G) served for comparison. Each assay result was normalized in comparison to the positive control which was defined as 100 % activity. The compounds 1-chloro-5-([1,4]diazepane-1-sulfonyl)-8-propoxy-isoquinoline (compound A), 1-chloro-5-(piperazin-1-sulfonyl)-8-methoxy-isoquinoline (compound B), 1-methyl-5-([1,4]diazepane-1-sulfonyl)-8-methoxy-isoquinoline (compound C), and 1-chloro-5-([1,4]diazepane-1-sulfonyl)-6-methoxy-isoquinoline (compound D) showed potent PIM inhibitory effect comparable to the on of 1-chloro-8-methoxy-Fasudil (compound G).

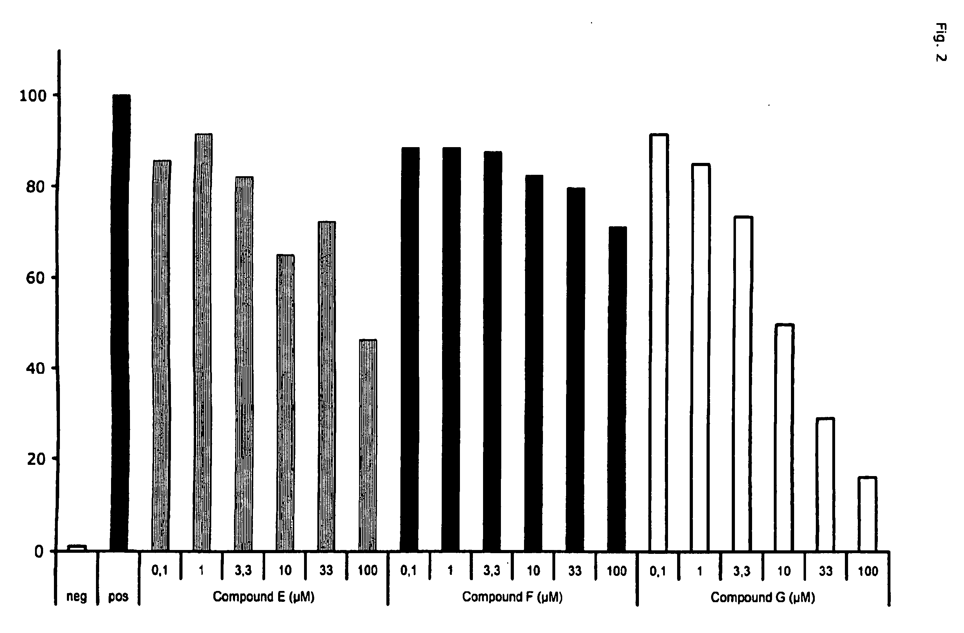

Fig. 2: The compound 5-([1,4]diazepane-1-sulfonyl)-8-methoxy-isoquinoline (compound E) exhibits only a weak inhibitory effect on PIM kinase.
   In vitro assays of PIM-1 kinase activity (2.5 ng) in the present of increasing concentrations of the compound E were performed as described within example 5. An assay without additional test compound served as positive control for the assay (pos) and an assay without PIM-1 kinase served as negative assay (neg). The compounds Fasudil (compound F) and potent PIM kinase inhibitor 1-chloro-8-methoxy-Fasudil (compound G) served for comparison. Each assay result was normalized in comparison to the positive control which was defined as 100 % activity. The compound 5-([1,4]diazepane-1-sulfonyl)-8-methoxy-isoquinoline (compound E) showed only weak PIM inhibitory effect slightly stronger than the one of Fasudil (compound F).

### Examples:

### Example 1: Synthesis of 1-chloro-5-([1,4]diazepane-1-sulfonyl)-8-propoxy-isoquinoline hydrochloride

### Synthesis of 8-propoxy-isoquinoline (2)

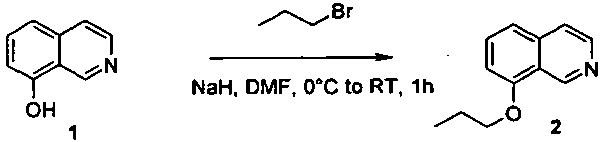

To a solution of 8-hydroxy-isoquinoline (1) (1.7 g, 11.7 mmol) in DMF (20 ml) at 0 °C was added NaH (940 mg, 23.4 mmol, 2.0 eq.). The mixture was stirred for 30 min. Bromopropane (1.6 ml, 17.6 mmol, 1.5 eq.) was then added. The reaction was allowed to warm to RT and was stirred for 1 h. The reaction was diluted with EtOAc and washed with water. The organic layer was dried over MgSO₄ and concentrated. The residue was purified by flash chromatography (cyclohexane/EtoAc 10/0 to 8/2) to afford 1.4 g (63 %) of 8-propoxy-isoquinoline (2) as red oil.

¹H NMR (300 MHz,DMSO-d₆) δ : 9.66 (s, 1H), 8.52 (d, J = 5.8 Hz, 1H), 8.17 (d, J = 7.1 Hz, 1H), 7.8 (t, J = 8.1 Hz, 1H), 7.65 (d, J = 8.2 Hz, 1H), 7.28 (d, J = 7.8 Hz, 1H), 4.02 (s, 3H).

### Synthesis of 8-propoxy-isoquinoline 2-oxide (3)

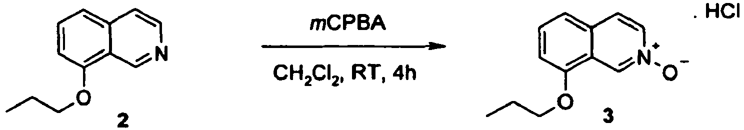

To a solution of 8-propoxy-isoquinoline (2) (1.4 g, 7.48 mmol) in CH₂Cl₂ (20 ml) at RT was added *m*CPBA (2.21 g, 8.97 mmol). The reaction was stirred at RT for 4 h. The reaction was dried over MgSO₄, filtered and the volume of solvent was reduced to 5 ml**.** 2 M HCl solution in Et₂O (10 ml) was added. The mixture was stirred at RT for 5 min and diluted with Et₂O. The precipitate was filtered to afford 1.34 g (74 %) of 8-propoxy-isoquinoline 2-oxide (3) as yellow solid.

¹H NMR (300 MHz,DMSO-d₆) δ : 9.17 (s, 1H), 8.42 (dd, J = 7.1, J = 1.7 Hz, 1H), 8.15 (d, J = 7.1 Hz, 1H), 7.76 (t, J = 8.1, 1H), 7.62 (d, J = 8.2 Hz, 1H), 7.25 (d, J = 7.9 Hz, 1H), 4.18 (t, J = 6.3 Hz, 2H), 1.97 (sex, J = 7.1 Hz, 2H), 1.14 (t, J = 7.3 Hz, 3H).

### Synthesis of 1-chloro-8-propoxy-isoquinoline (4)

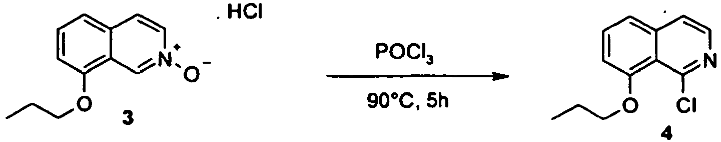

A solution of 8-propoxy-isoquinoline 2-oxide (3) (1.34 g, 5.59 mmol) in POCl₃ (25 ml) was heated at 90 °C for 5 h. The excess of POCl₃ was evaporated. The residue was carefully diluted with water and CH₂Cl₂ and then extracted with CH₂Cl₂. The combined organic layers were dried over MgSO₄ and concentrated. The residue was purified by flash chromatography (cyclohexane/EtOAc 10/0 to 8/2) to afford 350 mg (28 %) of 1-chloro-8-propoxy-isoquinoline (4) as a white solid.

**¹H** NMR (300 MHz,DMSO-d₆) δ: 8.20 (d, J = 5.6 Hz, 1H), 7.62 (t, J = 8.0 Hz, 1H), 7.50 (d,J=5.6Hz_{,} 1H), 7.36(d,J=8.13, 1H), 6.98(d,J=7.9Hz, 1H), 4.10 (t, J = 6.2 Hz, 2H), 1.99 (sex, J = 7.4 Hz, 2H), 1.17 (t, J = 7.4 Hz, 3H).

### Synthesis of 1-chloro-8-propoxy-isoquinoline-5-sulfonic acid (5)

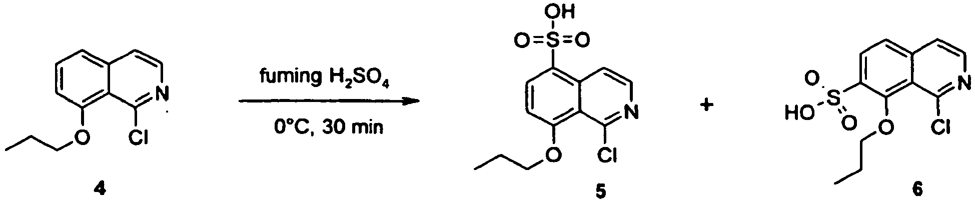

To a solution of fuming H₂SO₄ (20 % of SO₃, 4 ml) at 0 °C was added 1-chloro-8-propoxy-isoquinoline (4) portion wise (350 mg, 1.57 mmol). The reaction was stirred at 0 °C for 30 min. The mixture was poured on Et₂O. The precipitate was filtered and washed with Et₂O to afford 800 mg (quant.) a mixture of 1-chloro-8-propoxy-isoquinoline-5-sulfonic acid (5) plus 1-chloro-B-propoxy-isoquinoline-7-sulfonic acid (6) (3: 1) The crude product was used directly for the next step.

¹H NMR (300 MHz,DMSO-d₆) δ : 8.62 (d, J = 5.8 Hz, 1H), 8.23 (d, J = 5.8 Hz, 1H), 8.10 (d, J = 8.3 Hz, 1H), 7.11 (d, J = 8.3 Hz, 1H), 4.12 (t, J = 6.1 Hz, 2H), 1.86 (sex, J = 7.1 Hz, 2H), 1.08 (t, J = 7.3 Hz, 3H).

### Synthesis of 4-(1-chloro-8-propoxy-isoqulnoline-5-sulfonyl)-[1,4]diazepane-1-carboxylic acid benzyl ester (7)

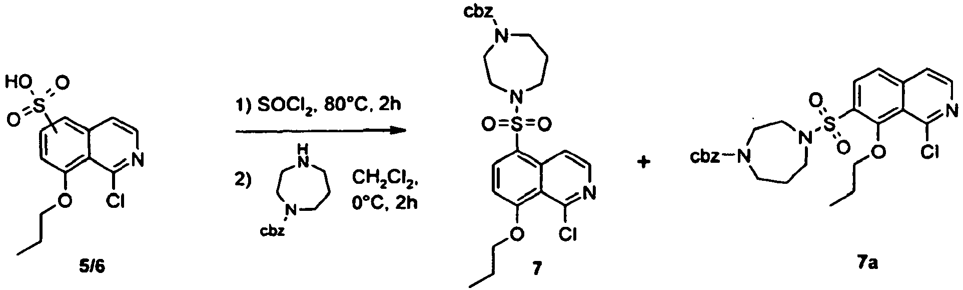

A solution of 1-chloro-8-propoxy-isoquinoline-5-sulfonic acid (5) and 1-chloro-8-propoxy-isoquinoline-7-sulfonic acid (6) (476 mg, 1.58 mmol) in SOCl₂ (5 ml) and DMF (0.5 ml) was heated at 80 °C for 2 h. The solvent was evaporated. The residue was quenched with H₂O and the solution was neutralized (pH = 8) by addition of a saturated NaHCO₃ solution. The mixture was extracted with CH₂Cl₂. The combined organic layers were poured drop wise to a solution of Cbz-homopiperazine (490 mg, 2.09 mmol) in CH₂Cl₂ (5 ml) at 0 °C. The reaction was stirred at 0 °C for 2 h. The reaction was washed with water. The organic layers were dried over MgSO₄, concentrated and purified by flash chromatography (cyclohexane/EtOAc 10/0 to 8/2) to afford 360 mg (44%) of 4-(1-chloro-8-propoxy-isoquinoline-5-sulfonyl)-[1,4]diazepane-1-carboxylic acid benzyl ester (7) as a yellow oil.

¹H NMR (300 MHz,DMSO-d₆) δ : 8.37-8.18 (m, 3H), 7.25 (m, 5H), 6.91 (d, J = 8.6 Hz, 1H), 5.02 (s, 2H), 4.13 (t, J = 6.1 Hz, 2H), 3.54-3.46 (m, 4H), 3.36-3.27 (m, 4H), 1.97-1.80 (m, 4H), 1.19 (t, J = 7.1 Hz, 3H).

### Synthesis of 1-chloro-5-([1,4]diazepane-1-sulfonyl)-6-methoxy-isoquinoline hydrochloride (8)

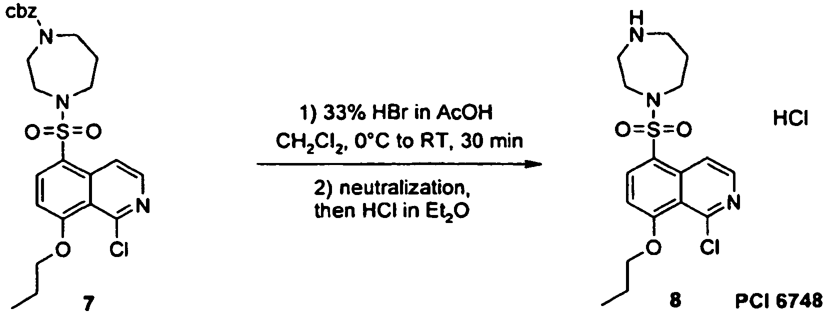

To a solution of 4-(1-chloro-8-propoxy-isoquinoline-5-sulfonyl)-[1,4]diazepane-1-carboxylic acid benzyl ester (7) (250 mg, 0.48 mmol) in CH₂Cl₂ (9 ml) at 0 °C was added 33 % HBr in AcOH. The reaction was stirred at RT for 30 min. The reaction was poured in water and neutralized (pH = 8) by addition of a saturated NaHCO₃ solution. The mixture was extracted with AcOEt. The organic layer was dried over MgSO₄ and concentrated. The residue was purified by flash chromatography (CH₂Cl₂/MeOH 100/0 to 85/15) The combined fractions were dissolved in MeOH and 2 M HCl in Et₂O (130 µl) was added. The mixture was concentrated to afford 95 mg (51 %) a white solid.

¹H NMR (300 MHz,DMSO-d₆) δ : 8.70 (broad s, 1H), 8.43 (d, J = 5.94 Hz, 1H), 8.34-8.28 (m, 2H), 7.31 (d, J = 8.8 Hz, 1H), 4.24 (t, J = 6.1 Hz, 2H), 3.58-3.55 (m, 2H), 3.42-3.40 (m, 2H), 3.14 (m, 4H), 1.96-1.87 (m, 4H), 1.10 (t, J = 7.3 Hz, 3H).

### Example 2: Synthesis of 1-chloro-8-methoxy-5-(piperazine-1-sulfonyl)-isoquinoline hydrochloride

### Synthesis of 8-methoxy-isoquinoline 2-oxide hydrochloride (10)

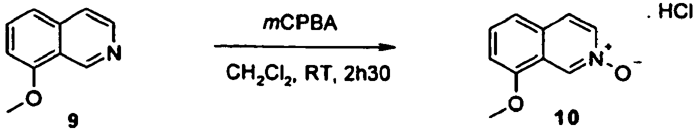

To a solution 8-methoxy-isoquinoline (9) (5.0 g, 31.4 mmol) in CH₂Cl₂ (100 ml) at RT was added *m*CPBA (9.3 g, 53.9 mmol). The reaction was stirred at RT for 2 h 30 min. The reaction was dried over MgSO₄ and the volume of solvent was reduced to 20 ml, and 22 ml of 2 M HCl solution in Et₂O was added. The mixture was stirred at RT for 5 min and diluted with Et₂O. The precipitate was filtered to afford 6.6 g (quant.) of 8-methoxy-isoquinoline 2-oxide hydrochloride (10) as yellow solid.

**¹H** NMR (300 MHz,DMSO-d₆) δ : 9.20 (s, 1H), 8.45 (dd, J = 7.1, 2 Hz, 1H), 8.17 (d, J = 7.1 Hz, 1H), 7.8 (t, J = 8.1 Hz, 1H), 7.65 (d, J = 8.2 Hz, 1H), 7.28 (d, J = 7.8 Hz, 1H), 4.02 (s, 3H, OCH₃).

### Synthesis of 1-chloro-8-methoxy-isoquinoline (11)

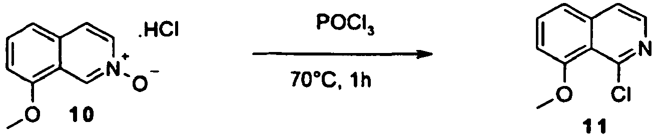

A solution of 8-methoxy-isoquinoline 2-oxide hydrochloride (10) (6.6 g, 31.2 mmol) in POCl₃ (30 ml) was heated at 70 °C for 1 h. The excess of POCl₃ was evaporated. The residue was carefully diluted with water and CH₂Cl₂ and then extracted with CH₂Cl₂. The combined organic layers were dried over MgSO₄ and concentrated. The residue was purified by flash chromatography (cyclohexane/EtOAc 10/0 to 8/2) to afford 2.8 g (46 %) of 1-chloro-8-methoxy-isoquinoline (11) as a white solid.

¹H NMR (300 MHz.DMSO-d₆) δ : 8.20 (d, J = 5.6 Hz, 1H), 7.59 (t, J = 4.3 Hz, 1H), 7.49 (d, J = 5.6 Hz, 1H), 7.37 (d, J = 7.6, 1H), 7.96 (d, J = 6.0 Hz, 1H), 4.00 (s, 3H, OCH₃).

### Synthesis of 1-chloro-8-methoxy-isoquinoline-5-sulfonic acid (12)

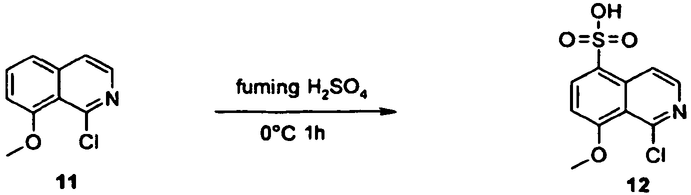

To a solution of fuming H₂SO₄ (20 % of **SO₃,** 10 ml) at 0 °C was added 1-chloro-8-methoxy-isoquinoline (11) portion wise (1.0 g, 5.16 mmol). The reaction was stirred at 0 °C for 1 h. The mixture was poured on Et₂O**.** The precipitate was filtered and washed with Et₂O to afford 1.5 g (quant.) of 1-chloro-8-methoxy-isoquinoline-5-sulfonic acid (12) as a yellow solid (mixture with inorganic salt).

¹H NMR (300 MHz,DMSO-d₆) δ : 8.63 (d, J = 5.9 Hz, 1H), 8.24 (d, J = 5.9 Hz, 1H), 8.13 (d, J = 8.3 Hz, 1H), 7.13 (d, J = 8.37 Hz, 1H), 3.96 (s, 3H, OCH₃).

### Synthesis of 4(-1-chloro-8-methoxy-isoquinoline-5-sulfonyl)-piperazine-1-carboxylic acid tert-butyl ester (13)

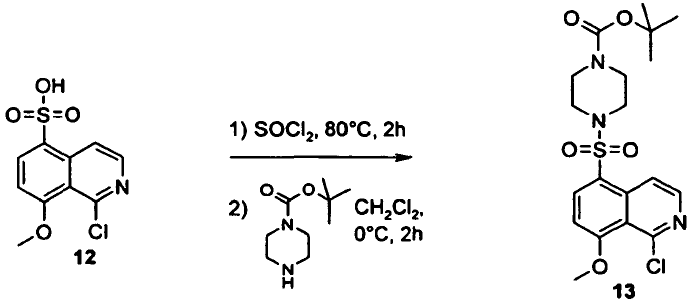

A solution of 1-chloro-8-methoxy-isoquinoline-5-sulfonic acid (12) (160 mg, 0.58 mmol) in SOCl₂ (5 ml) and DMF (0.1 ml) was heated at 80 °C for 2 h. The solvent was evaporated. The residue was dissolved with CH₂Cl₂ and ice was added and the solution was neutralized (pH = 6) by addition of a saturated NaHCO₃ solution. The mixture was extracted with CH₂Cl₂. The combined organic layers were poured drop wise to a solution of BOC-piperazine (273 mg, 1.46 mmol, 2.5 eq.) in CH₂Cl₂ (5 ml) at 0 °C. The reaction was stirred at 0 °C for 2 h. The reaction was washed with water. The organic layers were dried over Na₂SO₄, concentrated and purified by flash chromatography (cyclohexane/EtOAc 10/0 to 1/1) to afford 132 mg (51 %) of 4(-1-chloro-8-methoxy-isoquinoline-5-sulfonyl)-piperazine-1-carboxylic acid tert-butyl ester (13) as a white solid..

¹H NMR (300 MHz,CHCl₃-d) δ : 9.09 (broad s, 1H, NH), 8.47-8.37 (m, 3H), 7.42 (d, J = 8.8 Hz, 1H), 4.11 (s, 3H, OCH₃), 3.32-3.28 (m, 4H, 2*CH₂), 3.10 (broad s, 4H, 2*CH₂).

LC/MS = 100%, m/z = 442.2

### Synthesis of 1-chloro-8-methoxy-S-(piperazine-1-sulfonyl)-isoquinoline hydrochloride (14)

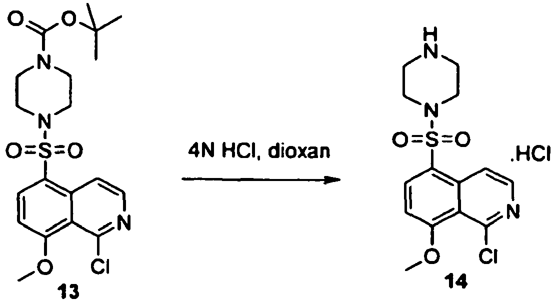

To a solution of 4(-1-chloro-8-methoxy-isoquinoline-5-sulfonyl)-piperazine-1-carboxylic acid tert-butyl ester (13) (132 mg, 0.30 mmol) in ethyl acetate (5 ml), was added 4 N HCl dioxan (4 ml), the reaction was stirred at room temperature for 4 h. The white precipitate was filtered and washed with Et₂O to afford 1-chloro-8-methoxy-5-(piperazine-1-sulfonyl)-isoquinoline hydrochloride (14) (110 mg) quant, as a white solid.

¹H NMR (300 MHz,DMSO-d₆) δ : 9.09 (broad s, 1H, NH), 8.47-8.37 (m, 3H), 7.42 (d, J = 8.8, 1H Hz,), 4.11 (s, 3H, OCH₃), 3.32-3.28 (m, 4H, 2*CH₂), 3.10 (broad s, 4H, 2*CH₂).

LC/MS = 100%

### Example 3: Synthesis of 5-([1.4]diazepane-1-sulfonyl)-8-methoxy-1-methyl-isoquinoline hyd rochloride

### Synthesis of 8-Methoxy-1-methyl-isoquinoline (15)

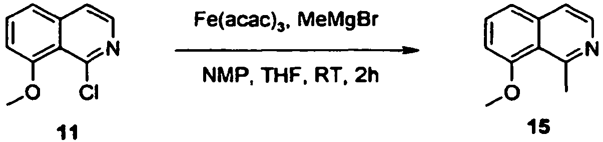

To a solution of 1-chloro-8-methoxy-isoquinoline (11) (205 mg, 1.06 mmol) in dry THF (5 ml) were added Fe (acac)₃ (38 mg , 0.106 mmol, 0.1 eq.), NMP (0.7 ml, 6.89 mmol, 6.5 eq.) and 3 M MeMgBr in Et₂O (0.46 mmol, 1.37 mmol, 1.3 eq.) at RT, the red mixture was stirred at RT for 2 h. The brown mixture was filtered over celite, quenched with a saturated NaCl solution and extracted with EtOAc. The organic phases were dried over Na₂SO₄ and concentrated. The residue was purified by flash chromatography (cyclohexane/EtOAc 10/0 to 8/2) to afford 170 mg g (55 %) of 8-Methoxy-1-methyl-isoquinoline (15) as a colorless oil.

¹H NMR (300 MHz,CHCl₃-d) δ : 8.33 (d, J = 5.7 Hz, 1H), 7.55 (t, J = 8.0 Hz, 1H), 7.42 (d, J = 5.7 Hz, 1H), 7.34 (d, J = 8.1 Hz, 1H), 6.90 (d, J = 7.9 Hz, 1H), 3.99 (s, 3H, OCH₃), 3.11 (s, 3H, CH₃).

### Synthesis of 8-methoxy-1-methyl-isoquinoline-5-sulfonic acid (16)

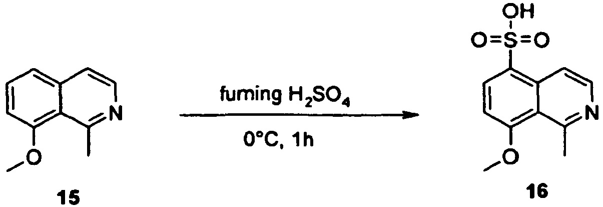

To a solution of fuming H₂SO₄ (20 % of SO₃, 2.0 ml) at 0 °C was added 8-Methoxy-1-methyl-isoquinoline (15) (170 mg, 0.98 mmol). The reaction was stirred at 0 °C for 1 h. The mixture was poured on Et₂O. The precipitate was filtered and washed with Et₂O to afford 320 mg (quant.) of 8-methoxy-1-methyl-isoquinoline-5-sulfonic acid (16) as a beige solid (mixture with inorganic salts).

¹H NMR (300 MHz,D₂O) δ : 8.64 (d, J = 7.1 Hz, 1H), 8.48 (d, J = 8.7 Hz, 1H), 8.22 (d, J = 7.1 Hz, 1H), 7.27 (d, J = 8.6 Hz, 1H), 4.05 (s, 3H, OCH₃), 3.65 (s, 3H, CH₃).

### Synthesis of 5-([1,4]diazepane-1-sulfonyl)-8-methoxy-1-methyl-isoquinoline (17)

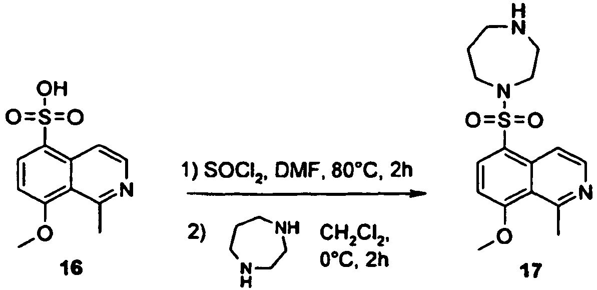

A solution of 8-methoxy-1-methyl-isoquinoline-5-sulfonic acid (16) (278 mg, 0.98 mmol) in SOCl₂ (5 ml) and DMF (0.1 ml) was heated at 80 °C for 2 h. The solvent was evaporated. The residue was dissolved CH₂Cl₂ and ice and the solution was neutralized (pH 6) by addition of a saturated NaHCO₃ solution. The mixture was extracted with CH₂Cl₂. The combined organic layers were poured drop wise to a solution of homopiperazine (392 mg, 3.92 mmol, 4.0 eq.) in CH₂Cl₂ (5 ml) at 0 °C. The reaction was stirred at 0 °C for 2 h. The reaction was washed with water. The organic layers were dried over MgSO₄, concentrated and purified by flash chromatography (CH₂Cl₂/MeOH 100/0 to 90/10) to afford 100 mg (30 %) of 5-([1,4]diazepane-1-sulfonyl)-8-methoxy-1-methyl-isoquinoline (17) as a yellow oil.

¹H NMR (300 MHz,CHCl₃-d) δ : 8.47 (d, J = 6.1 Hz, 1H), 8.31 (d, J = 8.5 Hz, 1H), 8.19 (d, J = 5.9 Hz, 1H), 6.91 (d, J = 8.6 Hz, 1H), 4.07 (s, 3H, OCH₃), 3.44-3.38 (m, 4H, 2*CH₂), 3.11 (s, 3H, CH₃), 2.97-2.91 (m, 4H, 2*CH₂), 1.86-1.77 (m, 2H, CH₂).

### Synthesis of 5-([1,4]diazepane-1-sulfonyl)-8-methoxy-1-methyl-isoquinoline hydrochloride (18)

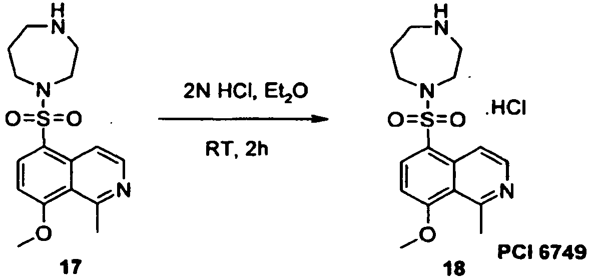

A solution of 5-([1,4]diazepane-1-sulfonyl)-8-methoxy-1-methyl-isoquinoline (17) (100 mg, 0.29 mmol) in 2 N HCl in Et₂O was stirred for 2 h at RT The white precipitate was filtered and washed with Et₂O to afford 5-([1,4]diazepane-1-sulfonyl)-8-methoxy-1-methyl-isoquinoline hydrochloride (18) as a yellow solid with 90 % yield.

¹H NMR (300 MHz,D₂O) δ : 8.59 (d, J = 7.1 Hz, 1H), 8.58 (d, J = 8.9 Hz, 1H), 8.29 (d, J = 7.1 Hz, 1H), 7.38 (d, J = 8.9 Hz, 1H), 4.12 (s, 3H, OCH₃), 3.67 (t, J = 5.1 Hz, , 2H, CH₂), 3.47 (t, J = 6.2 Hz, , 2H, CH₂), 3.34-3.31 (m, 4H, 2*CH₂), 3.23 (s, 3H, CH₃), 2.09-2.03 (m, 2H, CH₂).

### Example 4: Synthesis of 1-chloro-5-([1,4]diazepane-1-sulfonyl)-6-methoxy-isoquinoline hydrochloride

### Synthesis of 6-methoxy-isoquinoline 2-oxide hydrochloride (2):

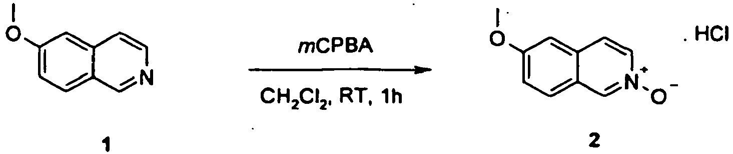

To a solution of 6-methoxy-isoquiniline (1) (2.5 g, 15.7 mmol) in CH₂Cl₂ (30 ml) at RT was added *m*CPBA (3.87 g, 18.1 mmol). The reaction was stirred at RT for 2 h 30 min. The volume of solvent was reduced to 15 ml and 22 ml of a saturated HCl solution of Et₂O was added. The mixture was stirred at RT for 5 min and diluted with Et₂O. The precipitate was filtered to afford 3.3 g (99%) of 6-methoxy-isoquinoline 2-oxide hydrochloride (2) as yellow solid.

¹H NMR (300 MHz,DMSO-d₆) δ : 9.61 (s, 1H), 8.53 (dd J = 7.2, 2 Hz, 1H), 8.22 (d, J = 9.1 Hz, ¹H), 8.21 (d, J = 7.2 Hz, 1H), 7.65 (s, 1H), 7.55 (dd, J = 9.1, 2 Hz, 1H), 3.97 (s, 3H).

### Synthesis of 1-chloro-6-methoxy-isoquinoline (3)

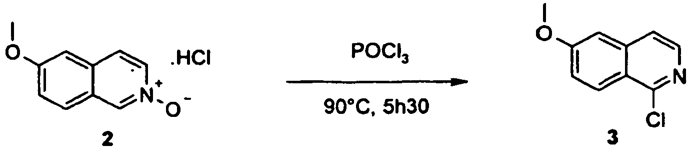

A solution of 6-methoxy-isoquinoline 2-oxide hydrochloride (2) (3.3 g, 15.6 mmol) in POCl₃ (25 ml) was heated at 90 °C for 5 h 30 min. The excess of POCl₃ was evaporated. The residue was carefully diluted with water and CH₂Cl₂ and then extracted with CH₂Cl₂. The combined organic layers were dried over MgSO₄ and concentrated. The residue was purified by flash chromatography (cyclohexane/EtOAc 10/0 to 8/2) to afford 1.69 g (56%) of 1-chloro-6-methoxy-isoquinoline (3) as a white solid.

**¹H** NMR (300 MHz,DMSO-d₆) δ : 8.19 (d, J = 5.6 Hz, 1H), 8.15 (d, J = 9.2 Hz, 1H), 7.75 (d, J = 5.6 Hz, 1H), 7.46 (s, 1H), 7.40 (d, J = 9.2 Hz, 1H), 3.92 (s, 3H).

### Synthesis of 1-Chloro-6-methoxy-isoquinoline-5-sulfonic acid (4)

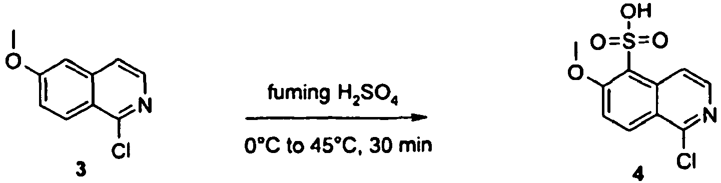

To a solution of fuming H₂SO₄ (20% of SO₃, 15 ml) at 0°C was added 1-chloro-6-methoxy-isoquinoline (3) portion wise (1.85 g, 9.55 mmol). The reaction was heated at 45 °C for 30 min. The mixture was poured on ice cold water and brought to pH 3 by addition of a saturated NaHCO₃ solution. The aqueous layer was washed with AcOEt (to remove impurities) and the aqueous phase was concentrated. The residue was triturated with iPrOH and the salts were filtered. The filtrate was concentrated to afford 4.0 g (quant.) of 1-Chloro-6-methoxy-isoquinoline-5-sulfonic acid (4) in mixture with inorganic salts.

¹H NMR (300 MHz,DMSO-d₆) δ: 9.12 (d, J = 6.3 Hz, 1H), 8.28 (d, J = 9.4 Hz, 1H), 8.10 (d, J = 6.3 Hz, 1H), 7.63 (d, J = 9.4 Hz, 1H), 3.91 (s, 3H).

### Synthesis of 1-chloro-5-([1,4]diazepane-1-sulfonyl)-6-methoxy-isoquinoline (5)

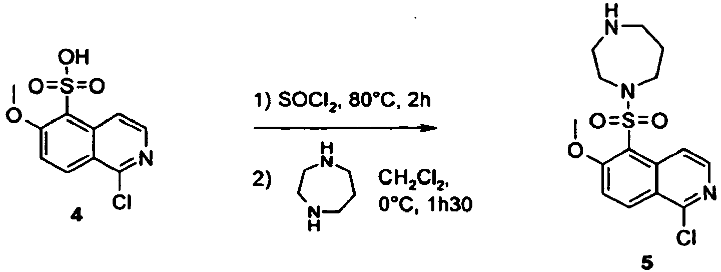

A solution of 1-Chloro-6-methoxy-isoquinoline-5-sulfonic acid (4) (2.0 g) in SOCl₂ (25 ml) and DMF (2 ml) was heated at 80 °C for 2 h. The solvent was evaporated. The residue was taken in H₂O and the solution was neutralized (pH 8) by addition of a saturated NaHCO₃ solution. The mixture was extracted with CH₂Cl₂. The combined organic layers were poured drop wise to a solution of homopiperazine (1.2 g, 12.0 mmol) in CH₂Cl₂ (10ml) at 0 °C. The reaction was stirred at 0 °C for 1 h 30 min. The reaction was washed with water. The organic layers were dried over MgSO₄, concentrated and purified by flash chromatography (CH₂Cl₂/MeOH 98/2 to 85/15) to afford 840 mg (49 %) of 1-chloro-S-([1,4]diazepane-1-sulfonyl)-6-methoxy-isoquinoline (5) as a yellow oil.

**¹H** NMR (300 MHz,DMSO-d₆) δ : 8.89 (d, J = 6.4 Hz, 1H), 8.56 (d, J = 9.5 Hz, 1H), 8.25 (d, J = 6.4 Hz, 1H), 7.44 (d, J = 9.5 Hz, 1H), 4.11 (s, 3H), 3.42 (m, 4H), 3.00 (m, 4H), 1.88 (m, 2H).

### Synthesis of 1-chloro-S-((1,4]diazepane-l-sulfonyl)-6-methoxy-isoquinoline hydrochloride (6)

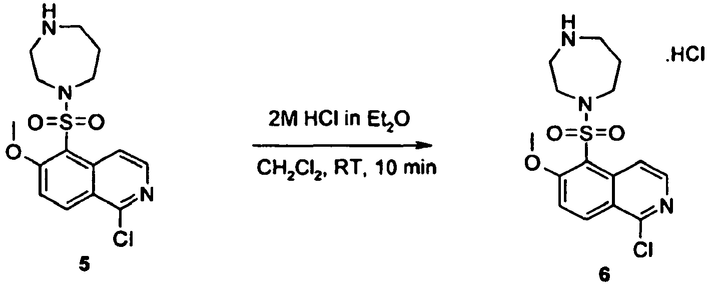

To a solution of 1-chloro-5-([1,4]diazepane-1-sulfonyl)-6-methoxy-isoquinoline (5) (1.19 g, 3.35 mmol) in CH₂Cl₂ (5 ml) was added 2 M HCl in Et₂O (1.68 ml, 3.35 mmol). The reaction was stirred at RT for 10 min. The reaction was diluted with Et₂O. The precipitate was filtered to afford 1.3 g (99%) of 1-chloro-5-([1,4]diazepane-1-sulfonyl)-6-methoxy-isoquinoline hydrochloride (6) as a yellow solid.

Mp : 219 - 221 °C

¹H NMR (300 MHz,DMSO-d₆) δ : 9.25 (broad s, NH.HCl), 8.72 (d, J = 6.3 Hz, 1H), 8.61 (d, J = 9.5 Hz, 1H), 8.33 (d, J = 6.3 Hz, 1H), 7.86 (d, J = 9.5 Hz, 1H), 4.15 (s, 3H), 3.69 - 6.65 (m, 2H), 3.38 - 3.31 (m, 2H), 3.23 - 3.17 (m, 4H), 2.07 - 1.99 (m, 2H).

### Example 5: In vitro assay for PIM-1 kinase inhibition

The in vitro assay for PIM-1 kinase inhibition was performed using the PIM-1 Kinase Assay Kit (Cell Signaling Technology Inc., Boston, USA) according to the manufacture's instruction. Briefly, 1.5 or 2.5 ng PIM-1 kinase has been dissolved in 50 µl assay buffer (25 mM Tris-Hcl (pH 7.5), 10 mM MgCl₂, 5 mM beta-glycerophosphate, 0.1 mM Na₃VO₄, 2 mM DTT, 200 µM ATP, and 1.5 µM peptide substrate) and test compound has been added to a final concentration in a range of 0.1 to 100 µM. After 30 minutes incubation at ambient temperature the reaction was stopped by adding 50 µl Stop buffer (50 mM EDTA, pH 8) per reaction. An aliquot of 25 µl per reaction was diluted each by 75 µl water and transferred to a 96-well streptavidin-coated plate. After 60 minutes incubation and three times washing with PBS, 100 µl/well primary antibody (Phospho-Bad (Ser112) antibody, 1:1000 diluted in PBS) was added. After further 2 h incubation and three times washing with PBS, an usual colorimetric ELISA assay was performed using a HRP labeled second antibody according to the manufacture's instructions. A PIM kinase reaction without addition of a test compound served as positive control (pos) and an assay without PIM kinase served as negative control (neg). Fasudil (compound F) which is exhibit at the most very weak PIM-1 inhibition and 1-chloro-8-methoxy-Fasudil (compound G) which is a very potent PIM-1 inhibitor served as further controls in each series of assays. The PIM-1 kinase inhibition of the test compounds (1-chloro-5-([1,4]diazepane-1-sulfonyl)-8-propoxy-isoquinoline (compound A), 1-chloro-5-(piperazin-1-sulfonyl)-8-methoxy-isoquinoline (compound B), 1-methyl-5-([1,4]diazepane-1-sulfonyl)-8-methoxy-isoquinoline (compound C), and 1-chloro-5-([1,4]diazepane-1-sulfonyl)-6-methoxy-isoquinoline (compound D)) was measured in a first series of assays, and the one of the compound 5-([1,4]diazepane-1-sulfonyl)-8-methoxy-isoquinoline (compound E) was measured in a second series of assays. Within each series of assays the measured PIM-1 kinase activity was normalized in comparison to the positive control (pos) which was defined as 100 % activity. The amount of PIM-1 kinase used in each series of assays was adapted in order to attain the same degree of inhibitions for the control compounds Fasudil and 1-chloro-8-methoxy-Fasudil to attain comparability of the inhibition results from separate series of assays. The compounds 1-chloro-5-([1,4]diazepane-1-sulfonyl)-8-propoxy-isoquinoline (compound A), 1-chloro-5-(piperazin-1-sulfonyl)-8-methoxy-isoquinoline (compound B), 1-methyl-5-([1,4]diazepane-1-sulfonyl)-8-methoxy-isoquinoline (compound C), and 1-chloro-5-([1,4]diazepane-1-sulfonyl)-6-methoxy-isoquinoline (compound D) revealed to be potent PIM-1 kinase inhibitors (Fig. 1), whereas the compound 5-([1,4]diazepane-1-sulfonyl)-8-methoxy-isoquinoline (compound E) exhibits at most a moderate PIM-1 kinase inhibition (Fig. 2). The table below summarizes the percentage of the remaining PIM-1 kinase activity in the presence of the test compounds in a final concentration of 100 µM.

| Percentage of the remaining PIM-1 kinase activity in presence of the test compounds (100 µM) | | | | | | | |
|---|---|---|---|---|---|---|---|
| Compound | A | B | C | D | E | F | G |
| % remaining PIM-1 kinase activity | 23 | 33 | 31 | 29 | 46 | 71 | 17 |

### Example 6: In vivo assays of PIM-1 kinase inhibitors

Efficacy of PIM inhibitors in cell culture can be tested as effects on proliferation of immortal cancer cell lines, e.g. the HeLa cell line and many others. Proliferation can be measured by counting cell density over time under a microscope, or by a number of biochemical assays. Invasion and spreading of cells can be assessed in soft agar tests, again using a large number of cancer cell lines. Induction of apoptosis in said cancer cells can be tested by assaying caspase 3 expression (e.g. by the Promega Caspase-glow assay). In vivo, anti cancer activity can be tested by transpainting cancer cell lines to animals, e.g. after immunosuuppression, and monitoring the growth of those tumours for example by a reporter gene such as luciferase, and bioimaging using life single photon imaging boxes, or radiological assays like magnetic resonance imaging (MRI) or Micro-CT. Volume of the tumours also can be assessed post mortem in those animals. Typically the experiment is done in two groups of animals, one receiving placebo, and one receiving the drug. Sample sizes are typically 20 per group. In those animals also the lifespan and mortality can be monitored, and provide a clinically meaningful endpoint. Typically, a broad range of concentrations and application modes is tested in vivo to find an optimal dose range.

## Claims

1. A compound of Formula I 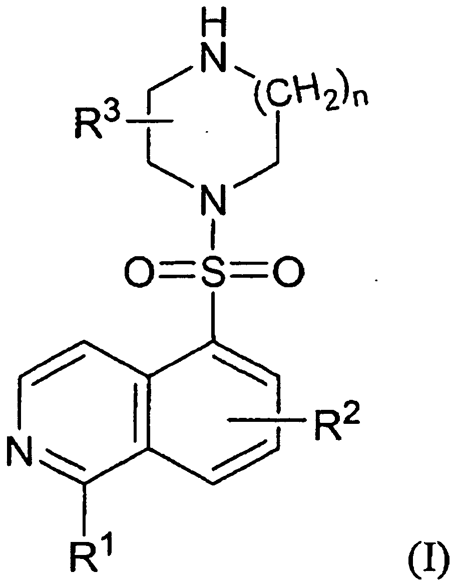 wherein
R¹ is a chlorine atom or a C₁₋₃ alkyl group,
R² is a C₁₋₃ alkoxy group at position 6 or 8 of the isoquinoline moiety,
R³ is a hydrogen or a C₁₋₃ alkyl group, and
n is 1 or 2.

2. A compound of Formula II 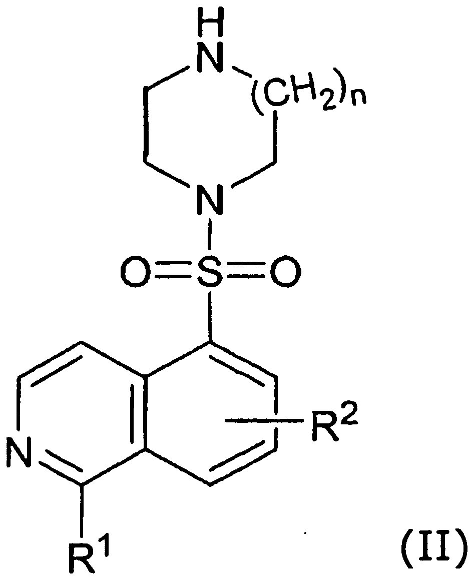 wherein
R1 is a chlorine atom or a C1-3 alkyl group,
R2 is a C1-3 alkoxy group at position 6 or 8 of the isoquinoline moiety, and
n is 1 or 2.

3. The compound of claim 1 or 2, wherein the compound 1-chloro-5-([1,4]diazepane-1-sulfonyl)-8-methoxy-isoquinoline is excluded.

4. The compound of claim 1 or 2, wherein the compounds according to the formula 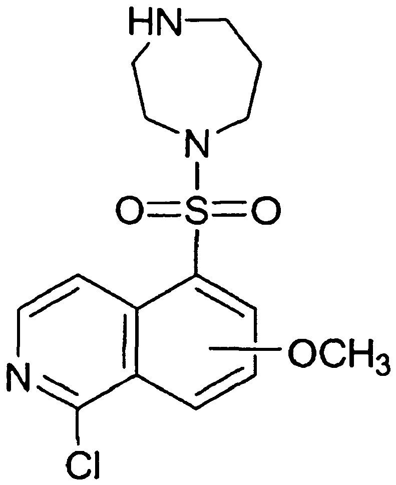 are excluded.

5. The compound of any one of claims 1 to 4, wherein R¹ is a chlorine atom.

6. The compound of any one of claims 1 to 4, wherein R¹ is a C₁₋₃ alkyl group.

7. The compound of claim 6, wherein R¹ is a methyl group.

8. The compound of claim 1 that is selected from the group consisting of 1-chloro-5-([1,4]diazepane-1-sulfonyl)-8-propoxy-isoquinoline, 1-chloro-5-(piperazin-1-sulfonyl)-8-methoxy-isoquinoline, 1-methyl-5-([1,4]diazepane-1-sulfonyl)-8-methoxy-isoquinoline, and 1-chloro-5-([1,4]diazepane-1-sulfonyl)-6-methoxy-isoquinoline.

9. A compound of any one of claims 1 to 8 for use in a method for inhibiting a PIM kinase, comprising contacting the PIM kinase with said compound, particularly wherein the PIM kinase is PIM-1 kinase.

10. An *in vitro* method for inhibiting a PIM kinase, comprising contacting the PIM kinase with a compound of any one of claims 1 to 8, particularly wherein the PIM kinase is PIM-1 kinase.

11. A compound of any one of claims 1 to 8 for use in a method of treating an oncological disease.

12. The compound for use of claim 11, wherein the oncological disease is one of the hematopoietic system, the liver or the prostate gland.

13. The compound for use of claim 11, wherein the oncological disease is selected from the group consisting of acute myelogenous leukemia (AML), acute lymphatic leukemia (ALL), chronic lymphatic leukemia (CLL), chronic myelogenous leukemia (CML), Hodgkin lymphadenoma, Non-Hodgkin lymphadenoma, and prostate carcinoma.

14. A pharmaceutical composition containing a compound of any one of claims 1 to 8.

15. The compound for use of claim 11 for use in a method for treating an oncological disease in a subject suffering from said disease, comprising administering to the subject a pharmaceutical effective amount of said compound.

## Patentansprüche

1. Verbindung der Formel I 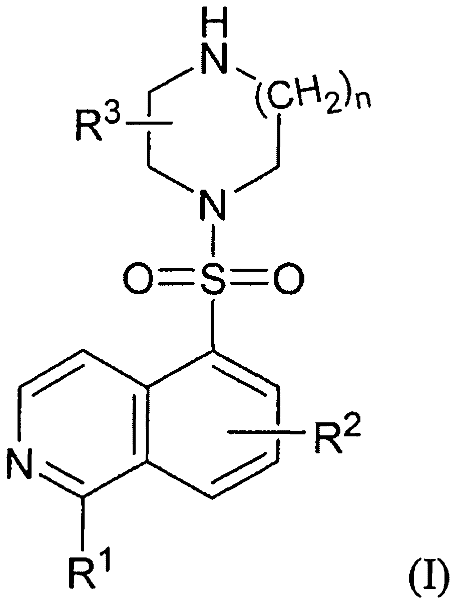 wobei
R¹ ein Chloratom oder eine C₁₋₃-Alkylgruppe ist,
R² eine C₁₋₃-Alkoxygruppe an Position 6 oder 8 der Isoquinolingruppierung ist,
R³ ein Wasserstoff oder eine C₁₋₃-Alkylgruppe ist, und
n 1 oder 2 ist.

2. Verbindung der Formel II 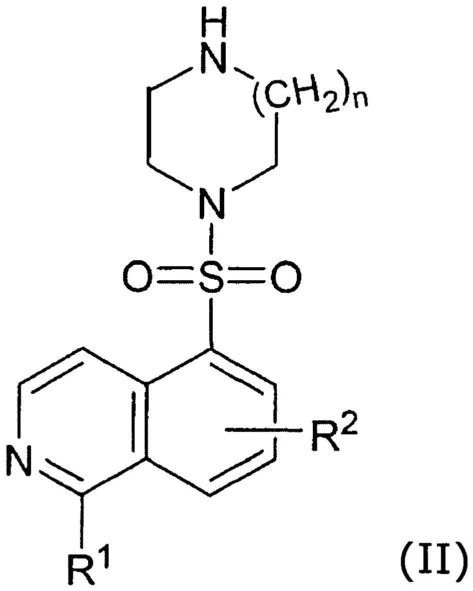 wobei
R¹ ein Chloratom oder eine C₁₋₃-Alkylgruppe ist,
R² eine C₁₋₃-Alkoxygruppe an Position 6 oder 8 der Isoquinolingruppierung ist, und
n 1 oder 2 ist.

3. Verbindung nach Anspruch 1 oder 2, wobei die Verbindung 1-Chlor-5-([1,4]diazepan-1-sulfonyl)-8-methoxyisoquinolin ausgeschlossen ist.

4. Verbindung nach Anspruch 1 oder 2, wobei die Verbindungen gemäß der Formel 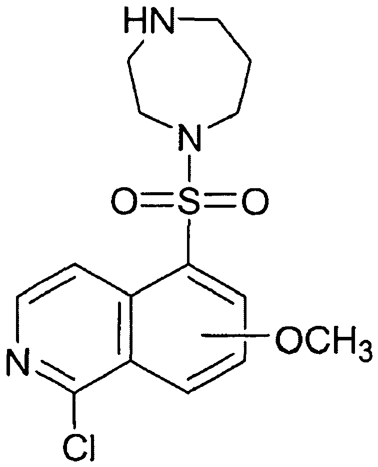 ausgeschlossen sind.

5. Verbindung nach einem beliebigen der Ansprüche 1 bis 4, wobei R¹ ein Chloratom ist.

6. Verbindung nach einem beliebigen der Ansprüche 1 bis 4, wobei R¹ eine C₁₋₃-Alkylgruppe ist.

7. Verbindung nach Anspruch 6, wobei R¹ eine Methylgruppe ist.

8. Verbindung nach Anspruch 1, die ausgewählt ist aus der Gruppe, bestehend aus 1-Chlor-5-([1,4]diazepan-1-sulfonyl)-8-propoxyisoquinolin, 1-Chlor-5-(piperazin-1-sulfonyl)-8-methoxyisoquinolin, 1-Methyl-5-([1,4]diazepan-1-sulfonyl)-8-methoxyisoquinolin und 1-Chlor-5-([1,4]diazepan-1-sulfonyl)-6-methoxyisoquinolin.

9. Verbindung nach einem beliebigen der Ansprüche 1 bis 8 zur Verwendung in einem Verfahren zum Inhibieren einer PIM-Kinase, umfassend In-Kontakt-Bringen der PIM-Kinase mit der Verbindung, insbesondere wobei die PIM-Kinase PIM-1-Kinase ist.

10. In-vitro-Verfahren zum Inhibieren einer PIM-Kinase, umfassend In-Kontakt-Bringen der PIM-Kinase mit einer Verbindung nach einem beliebigen der Ansprüche 1 bis 8, insbesondere, wobei die PIM-Kinase PIM-1-Kinase ist.

11. Verbindung nach einem beliebigen der Ansprüche 1 bis 8 zur Verwendung in einem Verfahren zum Behandeln einer onkologischen Krankheit.

12. Verbindung zur Verwendung nach Anspruch 11, wobei die onkologische Krankheit eine des blutbildenden Systems, der Leber oder der Vorsteherdrüse ist.

13. Verbindung zur Verwendung nach Anspruch 11, wobei die onkologische Krankheit ausgewählt ist aus der Gruppe, bestehend aus akuter myeloischer Leukämie (AML), akuter lymphatischer Leukämie (ALL), chronischer lymphatischer Leukämie (CLL), chronischer myeloischer Leukämie (CML), Hodgkin-Lymphom, Non-Hodgkin-Lymphom und Prostatakarzinom.

14. Pharmazeutische Zusammensetzung, enthaltend eine Verbindung nach einem beliebigen der Ansprüche 1 bis 8.

15. Verbindung zur Verwendung nach Anspruch 11 zur Verwendung in einem Verfahren zum Behandeln einer onkologischen Krankheit in einem Subjekt, das unter dieser Krankheit leidet, umfassend Verabreichen an das Subjekt einer pharmazeutisch wirksamen Menge der Verbindung.

## Revendications

1. Composé de formule I : 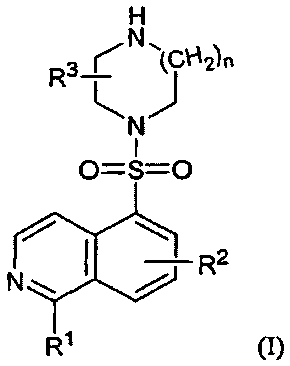 dans lequel
R¹ est un atome de chlore ou un groupe alkyle en C₁ à C₃,
R² est un groupe alcoxy en C₁ à C₃ en position 6 ou 8 du fragment isoquinoline,
R³ est un hydrogène ou un groupe alkyle en C₁ à C₃, et
n est 1 ou 2.

2. Composé de formule II : 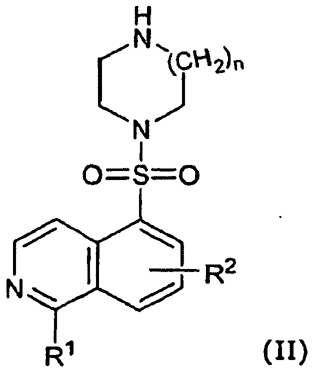 dans lequel
R¹ est un atome de chlore ou un groupe alkyle en C₁ à C₃,
R² est un groupe alcoxy en C₁ à C₃ en position 6 ou 8 du fragment isoquinoline, et
n est 1 ou 2.

3. Composé selon la revendication 1 ou 2, dans lequel le composé 1-chloro-5-([1,4]diazépane-1-sulfonyl)-8-méthoxy-isoquinoline est exclu.

4. Composé selon la revendication 1 ou 2, dans lequel les composés selon la formule 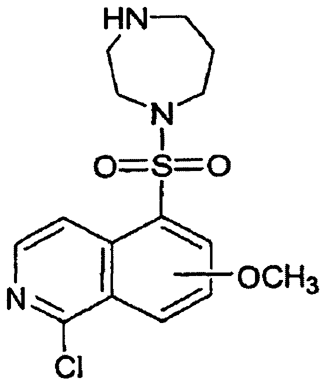 sont exclus.

5. Composé selon l'une quelconque des revendications 1 à 4, dans lequel R¹ est un atome de chlore.

6. Composé selon l'une quelconque des revendications 1 à 4, dans lequel R¹ est un groupe alkyle en C₁ à C₃.

7. Composé selon la revendication 6, dans lequel R¹ est un groupe méthyle.

8. Composé selon la revendication 1, qui est choisi dans le groupe constitué par la 1-chloro-5-([1,4]diazépane-1-sulfonyl)-8-propoxy-isoquinoline, la 1-chloro-5-(pipérazin-1-sulfonyl)-8-méthoxy-isoquinoline, la 1-méthyl-5-([1,4]diazépane-1-sulfonyl)-8-méthoxy-isoquinoline, et la 1-chloro-5-([1,4]diazépane-1-sulfonyl)-6-méthoxy-isoquinoline.

9. Composé selon l'une quelconque des revendications 1 à 8, pour une utilisation dans un procédé d'inhibition d'une kinase PIM, comprenant le contact de la kinase PIM avec ledit composé, la kinase PIM étant en particulier la kinase PIM-1.

10. Procédé d'inhibition *in vitro* d'une kinase PIM, comprenant le contact de la kinase PIM avec un composé selon l'une quelconque des revendications 1 à 8, la kinase PIM étant en particulier la kinase PIM-1.

11. Composé selon l'une quelconque des revendications 1 à 8, pour une utilisation dans un procédé de traitement d'une maladie oncologique.

12. Composé pour une utilisation selon la revendication 11, dans lequel la maladie oncologique est une maladie oncologique du système hématopoïétique, du foie ou de la prostate.

13. Composé pour une utilisation selon la revendication 11, dans lequel la maladie oncologique est choisie dans le groupe constitué de la leucémie myéloïde aiguë (AML), de la leucémie lymphatique aiguë (ALL), de la leucémie lymphatique chronique (CLL), de la leucémie myéloïde chronique (CML), du lymphome hodgkinien, du lymphome non hodgkinien et du carcinome de la prostate.

14. Composition pharmaceutique contenant un composé selon l'une quelconque des revendications 1 à 8.

15. Composé pour une utilisation selon la revendication 11, pour une utilisation dans un procédé de traitement d'une maladie oncologique chez un sujet souffrant de ladite maladie, comprenant l'administration au sujet d'une quantité pharmaceutique efficace dudit composé.
